# EUROPEAN PATENT APPLICATION

(11) **EP 1 362 867 A2**
(43) Date of publication of application: **19.11.2003**
(21) Application number: 03007983.4
(22) Date of filing: 10.04.2003
(51) Int. Cl.: C07K 14/47, C12N 15/11, A61K 38/17, A61K 48/00, G01N 33/48, C12N 15/12, C12N 15/86

(54) **Control of the ratio of LAP to LIP**

(30) Priority: 12.04.2002 JP 2002110197
(71) Applicant: Sumitomo Electric Industries, Ltd., Osaka-shi, Osaka 541-0041 (JP)
(72) Inventor: Hirai, Yohei, Sumitomo Electric Industries, Ltd., Yokohama-shi, Kanagawa 244-8588 (JP)
(74) Representative: Sternagel, Fleischer, Godemeyer & Partner Patentanwälte

(57) **Abstract**

The present invention provides recombinant gene vectors and nucleic acid constructs which are capable of decreasing, reducing or suppressing the transcription/translation of LIP (transcription inhibitor protein), and in some examples, without affecting the transcription/translation of LAP (transcription activator). Such vectors and constructs are used in methods for increasing the ratio of LAP to LIP activity in a cell and for treating and/or ameliorating the symptoms of a disease or condition associated with the ratio of LAP to LIP. The present invention also provides methods for screening for agents that are capable of modifying the ratio of LAP to LIP activity in a cell or individual.

SEQUENCE LISTING
<110> Sumitomo Electric Industries,Ltd.
<120> An agent for gene therapy
<130> FA2180A
<160> 8
<210> 1
<211> 894
<212> DNA
<213> Rat
<400> 1
atg cac cgc ctg ctg gcc tgg gac gca gca tgc ctc ccg ccg ccg ccc 48
Met His Arg Leu Leu Ala Trp Asp Ala Ala Cys Leu Pro Pro Pro Pro
1               5                   10                  15
gcc gcc ttt aga ccc atg gaa gtg gcc aac ttc tac tac gag ccc gac 96
Ala Ala Phe Arg Pro Met Glu Val Ala Asn Phe Tyr Tyr Glu Pro Asp
            20                  25                  30
tgc ctg gcc tac ggg gcc aag gcg gcc cgc gcc gcg ccg cgc gcc ccc 144
Cys Leu Ala Tyr Gly Ala Lys Ala Ala Arg Ala Ala Pro Arg Ala Pro
        35                  40                  45
gcc gcc gag ccg gcc atc ggc gag cac gag cgc gcc atc gac ttc agc 192
Ala Ala Glu Pro Ala Ile Gly Glu His Glu Arg Ala Ile Asp Phe Ser
    50                  55                  60
ccc tac ctg gag ccg ctc gcg ccc gcc gcc gcg gac ttc gcc gcg ccc 240
Pro Tyr Leu Glu Pro Leu Ala Pro Ala Ala Ala Asp Phe Ala Ala Pro
65                  70                  75                  80
gcg ccc gcg cac cac gac ttc ctt tcc gac ctc ttc gcc gac gac tac 288
Ala Pro Ala His His Asp Phe Leu Ser Asp Leu Phe Ala Asp Asp Tyr
                85                  90                  95
ggc gcc aag ccg agc aag aag ccg tcc gac tac ggt tac gtg agc ctc 336
Gly Ala Lys Pro Ser Lys Lys Pro Ser Asp Tyr Gly Tyr Val Ser Leu
            100                 105                 110
ggc cgc gcg ggc gcc aag gcc gca ccg ccc gcc tgc ttc ccg ccg ccg 384
Gly Arg Ala Gly Ala Lys Ala Ala Pro Pro Ala Cys Phe Pro Pro Pro
        115                 120                 125
cct ccc gcc gca ctc aag gcc gag ccg ggc ttc gaa ccc gcg gac tgc 432
Pro Pro Ala Ala Leu Lys Ala Glu Pro Gly Phe Glu Pro Ala Asp Cys
    130                 135                 140
aag cgc gcg gac gac gcg ccc gcc atg gcg gcc ggc ttc ccg ttc gcc 480
Lys Arg Ala Asp Asp Ala Pro Ala Met Ala Ala Gly Phe Pro Phe Ala
145                 150                 155                 160
ctg cgc gcc tac ctg ggc tac cag gcg acg ccg agc ggc agc agc ggc 528
Leu Arg Ala Tyr Leu Gly Tyr Gln Ala Thr Pro Ser Gly Ser Ser Gly
                165                 170                 175
agc ctg tcc acg tcg tcg tcg tcc agc ccg ccc ggg acg ccg agc ccc 576
Ser Leu Ser Thr Ser Ser Ser Ser Ser Pro Pro Gly Thr Pro Ser Pro
            180                 185                 190

gcc gac gcc aag gcc gcg ccc gcc gcc tgc ttc gcg ggg ccg ccg gcc 624
Ala Asp Ala Lys Ala Ala Pro Ala Ala Cys Phe Ala Gly Pro Pro Ala
        195                 200                 205
gcg ccc gcc aag gcc aag gcc aag aag gcg gtg gac aag ctg agc gac 672
Ala Pro Ala Lys Ala Lys Ala Lys Lys Ala Val Asp Lys Leu Ser Asp
    210                 215                 220
gag tac aag atg cgg cgc gag cgc aac aac atc gcg gtg cgc aag agc 720
Glu Tyr Lys Met Arg Arg Glu Arg Asn Asn Ile Ala Val Arg Lys Ser
225                 230                 235                 240
cgc gac aag gcc aag atg cgc aac ctg gag acg cag cac aag gtg ctg 768
Arg Asp Lys Ala Lys Met Arg Asn Leu Glu Thr Gln His Lys Val Leu
                245                 250                 255
gag ctg acg gcg gag aac gag cgg ctg cag aag aag gtg gag cag ctg 816
Glu Leu Thr Ala Glu Asn Glu Arg Leu Gln Lys Lys Val Glu Gln Leu
            260                 265                 270
tcg cga gag ctc agc acg ctg cgg aac ttg ttc aag cag ctg ccc gag 864
Ser Arg Glu Leu Ser Thr Leu Arg Asn Leu Phe Lys Gln Leu Pro Glu
        275                 280                 285
ccg ctg ctg gcc tcg gcg ggt cac tgc tag 894
Pro Leu Leu Ala Ser Ala Gly His Cys
    290                 295
<210> 2
<211> 297
<212> PRT
<213> Rat
<400> 2
Met His Arg Leu Leu Ala Trp Asp Ala Ala Cys Leu Pro Pro Pro Pro
1               5                   10                  15
Ala Ala Phe Arg Pro Met Glu Val Ala Asn Phe Tyr Tyr Glu Pro Asp
            20                  25                  30
Cys Leu Ala Tyr Gly Ala Lys Ala Ala Arg Ala Ala Pro Arg Ala Pro
        35                  40                  45
Ala Ala Glu Pro Ala Ile Gly Glu His Glu Arg Ala Ile Asp Phe Ser
    50                  55                  60
Pro Tyr Leu Glu Pro Leu Ala Pro Ala Ala Ala Asp Phe Ala Ala Pro
65                  70                  75                  80
Ala Pro Ala His His Asp Phe Leu Ser Asp Leu Phe Ala Asp Asp Tyr
                85                  90                  95
Gly Ala Lys Pro Ser Lys Lys Pro Ser Asp Tyr Gly Tyr Val Ser Leu
            100                 105                 110
Gly Arg Ala Gly Ala Lys Ala Ala Pro Pro Ala Cys Phe Pro Pro Pro
        115                 120                 125
Pro Pro Ala Ala Leu Lys Ala Glu Pro Gly Phe Glu Pro Ala Asp Cys
    130                 135                 140
Lys Arg Ala Asp Asp Ala Pro Ala Met Ala Ala Gly Phe Pro Phe Ala
145                 150                 155                 160

Leu Arg Ala Tyr Leu Gly Tyr Gln Ala Thr Pro Ser Gly Ser Ser Gly
                165                 170                 175
Ser Leu Ser Thr Ser Ser Ser Ser Ser Pro Pro Gly Thr Pro Ser Pro
            180                 185                 190
Ala Asp Ala Lys Ala Ala Pro Ala Ala Cys Phe Ala Gly Pro Pro Ala
        195                 200                 205
Ala Pro Ala Lys Ala Lys Ala Lys Lys Ala Val Asp Lys Leu Ser Asp
    210                 215                 220
Glu Tyr Lys Met Arg Arg Glu Arg Asn Asn Ile Ala Val Arg Lys Ser
225                 230                 235                 240
Arg Asp Lys Ala Lys Met Arg Asn Leu Glu Thr Gln His Lys Val Leu
                245                 250                 255
Glu Leu Thr Ala Glu Asn Glu Arg Leu Gln Lys Lys Val Glu Gln Leu
            260                 265                 270
Ser Arg Glu Leu Ser Thr Leu Arg Asn Leu Phe Lys Gln Leu Pro Glu
        275                 280                 285
Pro Leu Leu Ala Ser Ala Gly His Cys
    290                 295
<210> 3
<211> 1065
<212> DNA
<213> Human
<400> 3
atg caa cgc ctg gtg gcc tgg gac cca gca tgt ctc ccc ctg ccg ccg 48
Met Gln Arg Leu Val Ala Trp Asp Pro Ala Cys Leu Pro Leu Pro Pro
1               5                   10                  15
ccg ccg cct gcc ttt aaa tcc atg gaa gtg gcc aac ttc tac tac gag 96
Pro Pro Pro Ala Phe Lys Ser Met Glu Val Ala Asn Phe Tyr Tyr Glu
            20                  25                  30
gcg gac tgc ttg gct gct gcg tac ggc ggc aag gcg gcc ccc gcg gcg 144
Ala Asp Cys Leu Ala Ala Ala Tyr Gly Gly Lys Ala Ala Pro Ala Ala
        35                  40                  45
ccc ccc gcg gcc aga ccc ggg ccg cgc ccc ccc gcc ggc gag ctg ggc 192
Pro Pro Ala Ala Arg Pro Gly Pro Arg Pro Pro Ala Gly Glu Leu Gly
    50                  55                  60
agc atc ggc gac cac gag cgc gcc atc gac ttc agc ccg tac ctg gag 240
Ser Ile Gly Asp His Glu Arg Ala Ile Asp Phe Ser Pro Tyr Leu Glu
65                  70                  75                  80
ccg ctg ggc gcg ccg cag gcc ccg gcg ccc gcc acg gcc acg gac acc 288
Pro Leu Gly Ala Pro Gln Ala Pro Ala Pro Ala Thr Ala Thr Asp Thr
                85                  90                  95
ttc gag gcg gct ccg ccc gcg ccc gcc ccc gcg ccc gcc tcc tcc ggg 336
Phe Glu Ala Ala Pro Pro Ala Pro Ala Pro Ala Pro Ala Ser Ser Gly
            100                 105                 110
cag cac cac gac ttc ctc tcc gac ctc ttc tcc gac gac tac ggg ggc 384
Gln His His Asp Phe Leu Ser Asp Leu Phe Ser Asp Asp Tyr Gly Gly

        115                 120                 125
aag aac tgc aag aag ccg gcc gag tac ggc tac gtg agc ctg ggg cgc 432
Lys Asn Cys Lys Lys Pro Ala Glu Tyr Gly Tyr Val Ser Leu Gly Arg
    130                 135                 140
ctg ggg gct gcc aag ggc gcg ctg cac ccc ggc tgc ttc gcg ccc ctg 480
Leu Gly Ala Ala Lys Gly Ala Leu His Pro Gly Cys Phe Ala Pro Leu
145                 150                 155                 160
cac cca ccg ccc ccg ccg ccg ccg ccg ccc gcc gag ctc aag gcg gag 528
His Pro Pro Pro Pro Pro Pro Pro Pro Pro Ala Glu Leu Lys Ala Glu
                165                 170                 175
ccg ggc ttc gag ccc gcg gac tgc aag cgg aag gag gag gcc ggg gcg 576
Pro Gly Phe Glu Pro Ala Asp Cys Lys Arg Lys Glu Glu Ala Gly Ala
            180                 185                 190
ccg ggc ggc ggc gca ggc atg gcg gcg ggc ttc ccg tac gcg ctg cgc 624
Pro Gly Gly Gly Ala Gly Met Ala Ala Gly Phe Pro Tyr Ala Leu Arg
        195                 200                 205
gct tac ctc ggc tac cag gcg gtg ccg agc ggc agc agc ggg agc ctc 672
Ala Tyr Leu Gly Tyr Gln Ala Val Pro Ser Gly Ser Ser Gly Ser Leu
    210                 215                 220
tcc acg tcc tcc tcg tcc agc ccg ccc ggc acg ccg agc ccc gct gac 720
Ser Thr Ser Ser Ser Ser Ser Pro Pro Gly Thr Pro Ser Pro Ala Asp
225                 230                 235                 240
gcc aag gcc ccc ccg acc gcc tgc tac gcg ggg gcc ggg ccg gcg ccc 768
Ala Lys Ala Pro Pro Thr Ala Cys Tyr Ala Gly Ala Gly Pro Ala Pro
                245                 250                 255
tcg cag gtc aag agc aag gcc aag aag acc gtg gac aag cac agc gac 816
Ser Gln Val Lys Ser Lys Ala Lys Lys Thr Val Asp Lys His Ser Asp
            260                 265                 270
gag tac aag atc cgg cgc gag cgc aac aac atc gcc gtg cgc aag agc 864
Glu Tyr Lys Ile Arg Arg Glu Arg Asn Asn Ile Ala Val Arg Lys Ser
        275                 280                 285
cgc gac aag gcc aag atg cgc aac ctg gag acg cag cac aag gtc ctg 912
Arg Asp Lys Ala Lys Met Arg Asn Leu Glu Thr Gln His Lys Val Leu
    290                 295                 300
gag ctc acg gcc gag aac gag cgg ctg cag aag aag gtg gag cag ctg 960
Glu Leu Thr Ala Glu Asn Glu Arg Leu Gln Lys Lys Val Glu Gln Leu
305                 310                 315                 320
tcg cgc gag ctc agc acc ctg cgg aac ttg ttc aag cag ctg ccc gag 1008
Ser Arg Glu Leu Ser Thr Leu Arg Asn Leu Phe Lys Gln Leu Pro Glu
                325                 330                 335
ccc ctg ctc gcc tcc tcc ggc cac tgc tag 1065
Pro Leu Leu Ala Ser Ser Gly His Cys
            340                 345
<210> 4
<211> 345
<212> PRT

<213> Human
<400> 4
Met Gln Arg Leu Val Ala Trp Asp Pro Ala Cys Leu Pro Leu Pro Pro
1               5                   10                  15
Pro Pro Pro Ala Phe Lys Ser Met Glu Val Ala Asn Phe Tyr Tyr Glu
            20                  25                  30
Ala Asp Cys Leu Ala Ala Ala Tyr Gly Gly Lys Ala Ala Pro Ala Ala
        35                  40                  45
Pro Pro Ala Ala Arg Pro Gly Pro Arg Pro Pro Ala Gly Glu Leu Gly
    50                  55                  60
Ser Ile Gly Asp His Glu Arg Ala Ile Asp Phe Ser Pro Tyr Leu Glu
65                  70                  75                  80
Pro Leu Gly Ala Pro Gln Ala Pro Ala Pro Ala Thr Ala Thr Asp Thr
                85                  90                  95
Phe Glu Ala Ala Pro Pro Ala Pro Ala Pro Ala Pro Ala Ser Ser Gly
            100                 105                 110
Gln His His Asp Phe Leu Ser Asp Leu Phe Ser Asp Asp Tyr Gly Gly
        115                 120                 125
Lys Asn Cys Lys Lys Pro Ala Glu Tyr Gly Tyr Val Ser Leu Gly Arg
    130                 135                 140
Leu Gly Ala Ala Lys Gly Ala Leu His Pro Gly Cys Phe Ala Pro Leu
145                 150                 155                 160
His Pro Pro Pro Pro Pro Pro Pro Pro Pro Ala Glu Leu Lys Ala Glu
                165                 170                 175
Pro Gly Phe Glu Pro Ala Asp Cys Lys Arg Lys Glu Glu Ala Gly Ala
            180                 185                 190
Pro Gly Gly Gly Ala Gly Met Ala Ala Gly Phe Pro Tyr Ala Leu Arg
        195                 200                 205
Ala Tyr Leu Gly Tyr Gln Ala Val Pro Ser Gly Ser Ser Gly Ser Leu
    210                 215                 220
Ser Thr Ser Ser Ser Ser Ser Pro Pro Gly Thr Pro Ser Pro Ala Asp
225                 230                 235                 240
Ala Lys Ala Pro Pro Thr Ala Cys Tyr Ala Gly Ala Gly Pro Ala Pro
                245                 250                 255
Ser Gln Val Lys Ser Lys Ala Lys Lys Thr Val Asp Lys His Ser Asp
            260                 265                 270
Glu Tyr Lys Ile Arg Arg Glu Arg Asn Asn Ile Ala Val Arg Lys Ser
        275                 280                 285
Arg Asp Lys Ala Lys Met Arg Asn Leu Glu Thr Gln His Lys Val Leu
    290                 295                 300
Glu Leu Thr Ala Glu Asn Glu Arg Leu Gln Lys Lys Val Glu Gln Leu
305                 310                 315                 320
Ser Arg Glu Leu Ser Thr Leu Arg Asn Leu Phe Lys Gln Leu Pro Glu
                325                 330                 335
Pro Leu Leu Ala Ser Ser Gly His Cys
            340                 345

<210> 5
<211> 37
<212> DNA
<213> Artificial Sequence
<220>
<223> Oligo DNA
<400> 5
gggggatccc gccatggaag tggccaactt ctactac 37
<210> 6
<211> 36
<212> DNA
<213> Artificial Sequence
<220>
<223> Oligo DNA
<400> 6
atatgctagc gcgggcgcgt cgtccgcgcg cttgca 36
<210> 7
<211> 36
<212> DNA
<213> Artificial Sequence
<220>
<223> Oligo DNA
<400> 7
atatgctagc ggccggcttc ccgttcgccc tgcgcg 36
<210> 8
<211> 36
<212> DNA
<213> Artificial Sequence
<220>
<223> Oligo DNA
<400> 8
atatgctagc agtgacccgc cgaggccagc agcggc 36

## Description

### TECHNICAL FIELD

The present invention provides compositions and methods for controlling the ratio of LAP to LIP in a cell. In particular, the present invention provides recombinant vectors encoding LAP (transciptional activator) activity wherein expression of LIP activity is inhibited, and wherein expression of LIP activity is inhibited such that LIP does not down-regulate LAP activity, as well as oligonucleotides and antisense nucleic acid that reduce expression of LIP activity. The present invention also provides methods for screening whether an agent modulates the ratio of the expression levels of LAP to LIP which are expressed from the C/EBPβ gene.

### BACKGROUND OF THE INVENTION

In spite of extensive medical research and numerous advances, cancer remains the second leading cause of death in the United States. Cancer formation is accompanied by continuous gene defects. Most of the cases that have been reported as gene defects were caused due to the acquisition of transformation function of predominant cancer genes or the loss of cancer suppressing function of cancer suppressor genes such as p53. The symptoms of cancer may be ameliorated and/or treated by the introduction of predominant negative variants of cancer genes or cancer suppressor genes into cancer cells by using, for example, p53 integrated retrovirus vector. Alavi J.B. et al. (2001, *Expert. Opin. Biol. Ther.* 1:239-252).

Introduction of a cytokine gene into cells or tissues for treatment of cancer has also been attempted. For example, the following attempts were made: introducing a cytokine gene into lymphocytes (e.g., lymphokine activated killer cells (LAK), cytotoxic T lymphocytes (CTL), and tumor-infiltrating lymphocytes (TIL)) and activating lymphocytes by cytokine secreted from the transduced cells so as to enhance passive immunity; or enhancing anti-tumor activity at tumor sites by directly introducing IFN or TNF gene having an anti-tumor activity into TILs. Treisman J. et al. (1994, *Cell Immunol*. 156: 448-457) and Hwu et al. (1993, *J. Immumol.* 150:4104-4115).

Furthermore, an attempt has been reported wherein a cytokine gene is introduced into tumor cells by retrovirus vectors, etc., and tumor-specific immune cells of host are induced by using the transduced cells as a tumor vaccine. Adris S. et al (2000, *Cancer Res.* 60:6696-6703) and Hiroishi K et al. (1999, *Gen Ther*. 12: 1988-1994).

The CCAAT/enhancer binding protein β (C/EBPβ) gene has been implicated in many developmental processes. Hirai et al. (2001, *J. of Cell Biol.* Vol. 153: 785-794). Two transcription initiation sites exist in the nucleotide sequence of the C/EBPβ gene, and LAP (transcriptional activator) and LIP (transcriptional repressor) are generated by translation from each of the transcription sites. Descombes et al. (1991, *Cell,* Vol. 67, 569-579). It has further been reported that, as the LAP/LIP ratio increases, the transcription activity of a target gene is enhanced, and the LAP/LIP ratio increases at a final stage in the differentiation of rat liver. Descombes et al., supra. Buck et al. (1994, *EMBO Journal* Vol.13, No.4, pp.851-860) disclose that the differentiation and the resting state of hepatic cells may be modulated by the LAP/LIP ratio.

Hirai et al., supra, report that when cells were treated with epimorphin, a protein which is expressed on the surface of myoepithelial cells and fibroblast cells of the mammary gland and is involved in the morphogenesis of the mammary gland, the expression of C/EBPβ increases and the encoded LAP/LIP ratio is varied.

In spite of advances in the treatment of symptoms of cancer, there remains a need for methods of screening for agents that can treat and/or ameliorate the symptoms of cancer as well as methods for treating and/or ameliorating the symptoms.

All patents and publications disclosed herein are hereby incorporated by reference in their entirety.

### SUMMARY OF THE INVENTION

The invention disclosed herein provides compositions and methods for controlling the ratio of LAP activity to LIP activity in a cell or individual. The present invention provides recombinant vectors encoding LAP (transciptional activator) activity wherein expression of LIP activity is inhibited, and wherein expression of LIP activity is inhibited such that LIP does not down-regulate LAP activity. In some examples, a recombinant vector comprises a nucleotide sequence for part or all of the CCAAT/enhancer binding protein β (C/EBPβ) gene wherein said part or all of the C/EBPβ gene comprises a nucleotide sequence around the initiation codon, ATG, of the LIP transcription inhibitor protein, and wherein the nucleotide sequence comprises a mutation around the initiation codon, ATG, of said LIP transcription inhibitor protein. In other examples, the mutation around the initiation codon is a substitution of ATG with a codon encoding another amino acid, wherein the substituted codon is not TTG. In other examples, the mutation is a substitution of ATG with a codon encoding an amino acid from the group consisting of Ala, Gly, and Pro. In yet other examples, the mutation is a substitution of ATG with a codon encoding an amino acid, Arg. In some examples, the recombinant vector is a viral vector, including a retrovirus vector, an adenovirus vector, of an adeno-associated virus vector. In other examples, the present invention provides viral particles comprising a viral vector of the present invention.

The present invention also provides variant LAP polypeptides comprising a substitution of the initiating codon, ATG, of LIP with another codon, wherein the substituted codon is not TTG and in some examples, is not a translational stop codon. In some examples, a variant LAP polypeptide comprises a substitution of the initiating codon ATG of LIP with a codon encoding Ala, Gly, Pro or Arg. The present invention also provides isolated nucleic acid encoding such polypeptides. The present invention also provides oligonucleotides comprising a nucleotide sequence of about 10 to about 100 nucleotides in length from around the initiation codon, ATG, of LIP (transcription inhibitor protein) in the nucleotide sequence of a C/EBPβ gene, or a complementary sequence thereof, wherein said oligonucleotide, or a complementary sequence thereof, is capable of reducing LIP expression. In some examples, the oligonucleotide is from about 10 to 80 nucleotides in length. In other examples, the oligonucleotide is from about 15 to about 50 nucleotides in length.

The present invention also provides host cells, compositions and kits comprising a recombinant vector, oligonucleotide or variant LAP polypeptide (or isolated nucleic acid encoding said polypeptide) of the present invention as well as methods of making such.

The present invention also provides methods for modifying the ratio of the expression level of LAP (transcription activator) to LIP (transcription inhibitor protein) in a cell, comprising contacting the cell with a recombinant vector of the present invention or an oligonucleotide of the present invention or a variant LAP polypeptide of the present invention under suitable conditions. In some examples, the cell is a cancer cell, such as a breast or liver cancer cell. The present invention also provides methods for treating and/or ameliorating the symptoms of a disease and/or condition associated with an abnormal ratio of the expression level of LAP (transcription activator) to LIP (transcription inhibitor protein) in an individual comprising administering to the individual a recombinant vector, or an oligonucleotide, or a variant LAP polypeptide of the present invention. In some examples, the disease or condition is cancer. The present invention also provides methods of screening whether an agent modulates the ratio of the expression level of LAP (transcription activator) to LIP (transcription inhibitor protein) which are expressed from a C/EBPβ gene comprising the step of contacting the C/EBPβ gene with said agent and measuring the ratio of LAP expression to LIP expression. In some examples, the agent is a low molecular weight compound; in other examples, the agent is an extract from a naturally occurring substance. In yet other examples, the ratio is measured by Northern blot. In some examples, a mammalian cell comprises said C/EBP 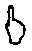 gene. In some examples, the method further comprises identifying an agent that increases the expression level of LAP in a cell or identifying an agent that decreases the expression level of LIP in a cell.

The present invention also relates to the use of a recombinant vector of the present invention in the manufacture of a medicament for the treatment and/or amelioration of symptoms of cancer. The present invention also relates to the use of a variant LAP polypeptide of the present invention in the manufacture of a medicament for the treatment and/or amelioration of symptoms of cancer. The present invention also relates to us of an oligonucleotide of the present invention in the manufacture of a medicament for the treatment and/or amelioration of symptoms of cancer.

### BRIEF DESCRIPTION OF THE SEVERAL VIEWS OF THE DRAWING(S)

Fig. 1 illustrates morphologies of g6 cells and g6 LAP cells.

Fig. 2 shows the results of induction of expression of E-cadherin by mutation- introduced LAP.

### DETAILED DESCRIPTION OF THE INVENTION

The invention disclosed herein provides compositions and methods for controlling the ratio of LAP to LIP in a cell. The present invention provides compositions and methods for modifying the ratio of LAP to LIP in a cell and in some examples, increasing the ratio of LAP to LIP. The invention disclosed herein provides compositions and methods of treating and/or ameliorating the symptoms of diseases and/or conditions associated with the ratio of transcriptional activator LAP to transcriptional repressor LIP from the CCAAT/enhancer binding protein 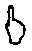 (C/EBP 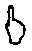 ) gene. The present invention also provides compositions and methods for screening for agents which modify the ratio of LAP to LIP.

The present invention provides recombinant vectors encoding the LAP (transcriptional activator) activity wherein expression of LIP activity is inhibited. In some examples, the present invention provides recombinant vectors encoding the LAP (transcriptional activator) activity wherein expression of LIP activity is inhibited, and wherein expression of LIP activity is inhibited such that the LIP activity does not down-regulate LAP activity. The present invention provides isolated nucleic acid encoding variant forms of LAP wherein the initiating codon ATG of LIP has been substituted with another codon, wherein the substituted codon is not TTG and, in some examples, is not a stop codon. The present invention provides variant LAP polypeptides wherein the initiating codon ATG of LIP has been substituted with another codon, wherein the substituted codon is not TTG and, in some examples, is not a stop codon. In some examples, such a variant LAP polypeptide exhibits oncolytic activity.

In particular, the present invention provides recombinant vectors comprising nucleic acid for part or all of the C/EBP 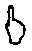 gene wherein said nucleic acid comprises a mutation in the nucleotide sequence around the initiating codon of LIP in the nucleotide sequence of C/EBP 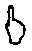 . In some examples, such recombinant vectors exhibit oncolytic activity and can be used for gene therapy, that is, for administration or delivery to a cell or individual subject to a disease or condition associated with the ratio of LAP to LIP. In particular, recombinant vectors of the present invention can be used for administration or delivery of a recombinant vector expressing LAP activity to a cell. Such recombinant vectors can be used in methods for increasing the ratio of LAP to LIP activity in a cell; in methods for treating and/or ameliorating the symptoms of diseases and/or conditions associated with the ratio of LAP to LIP in an individual subject to a disease and/or condition associated with the ratio of LAP to LIP; and in methods for screening for agents which modify the ratio of LAP to LIP. The present invention also provides oligonucleotides, antisense nucleic acid and interfering RNAs that are capable of decreasing, reducing or suppressing LIP expression.

The present invention disclosed herein provides methods for modifying the ratio of the expression level of LAP to LIP in a cell comprising contacting the cell with a recombinant vector of the present invention, or an oligonucleotide, antisense nucleic acid or interfering RNA of the present invention that is capable of decreasing, reducing or suppressing expression of LIP. In some examples, the cell is contacted with a recombinant vector of the present invention, or an oligonucleotide, antisense nucleotide or interfering RNA of the present invention in combination with epimorphin. The present invention also provides methods for treating and/or ameliorating the symptoms of diseases and/or conditions associated with the ratio of the expression level of LAP to LIP in an individual, such as cancer, comprising administering a recombinant vector of the present invention, or oligonucleotide, antisense nucleic acid or interfering RNA of the present invention, alone or in combination with other therapies or in combination with epimorphin, to an individual subject to cancer.

The present invention also provides methods for screening whether an agent modulates the ratio of the expression levels of LAP to LIP which are expressed from the C/EBPβ gene. The present invention also provides methods for determining if a particular cancer or malignant cell is susceptible to treatment with a recombinant vector of the present invention or an oligonucleotide, antisense nucleic acid or interfering RNA of the present invention. Accordingly, the present invention also provides kits comprising a recombinant vector of the present invention or an oligonucleotide, antisense nucleic acid or interfering RNA of the present invention. In some examples, a kit of the present invention comprises a recombinant vector that is used to test susceptibility of a cancer cell to treatment.

The present invention also provides compositions comprising a recombinant vector of the present invention or an oligonucleotide, antisense nucleic acid or interfering RNA of the present invention, as well as methods of making recombinant vector of the present invention or an oligonucleotide, antisense nucleic acid or interfering RNA of the present invention.

The practice of the present invention employs, unless otherwise indicated, conventional molecular biology, virology, microbiology, immunology, and recombinant DNA techniques which are within the skill of the art. These techniques are fully explained in the literature. See, *e.g.,* Maniatis et al., *Molecular Cloning: A Laboratory Manual* (1982); *DNA Cloning: A Practical Approach,* vols. I & II (D. Glover, ed.); *Oligonucleotide Synthesis* (N. Gait, ed. (1984)); *Nucleic Acid Hybridization* (B. Hames & S. Higgins, eds. (1985)); *Transcription and Translation* (B. Hames & S. Higgins, eds. (1984)); *Animal Cell Culture* (R. Freshney, ed. (1986)); Perbal, *A Practical Guide to Molecular Cloning* (1984); Ausubel, *et al., Current Protocols In Molecular Biology,* John Wiley & Sons (1987, 1988, 1989, 1990, 1991, 1992, 1993, 1994, 1995, 1996); and Sambrook *et al., Molecular Cloning: A Laboratory Manual* (2^{nd} Edition); vols. I, II & III (1989).

I. CCAAT/enhancer binding protein 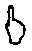 (C/EBP 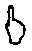 ); LAP and LIP

CCAAT/enhancer binding proteins ("C/EBP") comprise a class of enhancer binding proteins (EBPs) whose members are capable of preferentially recognizing and binding a CCAAT sequence motif (such as is found in the transferrin and ApoB genes), an enhancer core sequence motif, or the enhancer regions of several viral promoters (Landschultz, W. H. et al., *Genes Dev.* 2:786-800 (1989); Brunel, F. et al., *J. Biol. Chem.* 263:10180-10185 (1988); Metzger, S. et al., *J. Biol. Chem.* 265:9978-9983 (1990)).

C/EBPβ genes derived from mammals are known and disclosed in, for example, Descombes et al. (1990, *Genes Dev.* Vol. 4, pgs. 1541-1551) and Akira et al. (1990, *EMBO J.* 9, 1897-1906). The nucleotide sequence and amino acid sequence of rat-derived C/EBPβ gene are described as SEQ ID NOS: 1 and 2, respectively. The nucleotide sequence and amino acid sequence of human-derived C/EBPβ gene are described as SEQ ID NOS: 3 and 4, respectively.

The C/EBPβ gene has 2 translation products, that is, LAP (transcription activator) and LIP (transcription repressor). The existence of 2 types of translation products is attributable to the existence of 2 or more transcription initiation sites in the nucleotide sequence of a C/EBPβ (CCAAT/enhancer binding protein β) gene. Either LAP (transcription activator) or LIP (transcription repressor) is generated by initiating transcription/translation from either of these transcription initiation sites.

LAP is a protein which has a transcription control site and a DNA binding site from the N-terminal side, while LIP is a protein which has only a DNA binding site of LAP and has no transcription control site. Both proteins have an identical frame, that is, LIP shares with LAP the C-terminal 145 amino acids (Buck et al, supra). Without being bound by theory, Descombes et al. (1991, *Cell*, vol. 67: pages 569-579) suggest that LAP and LIP are translated from the same mRNAs by a leaky ribosome scanning mechanism. The initiation codon, ATG, of LIP (transcription inhibitor protein) corresponds to the 457th to 459th nucleotides in the nucleotide sequence of SEQ ID NO: 1, and to the 595th to 597th nucleotides in the nucleotide sequence of SEQ ID NO: 3.

Buck et al., *supra*, report that LAP expression and S-phase are mutually exclusive in hepatoma cells. They found that LAP inhibits hepatoma cell proliferation before the G₁/S boundary and prevents cells from entering S-phase. Descombes et al. (1991, *Cell* vol. 67:569-579) disclose that LAP and LIP share the 145 C-terminal amino acids that contain the basic DNA-binding domain and the leucine zipper dimerization helix. Buck et al., *supra,* disclose that the integrity of the LAP leucine zipper is required to prevent hepatoma cells from entering S-phase. Buck et al., *supra,* also disclose that LIP (which lacks the N-terminal activation domain of LAP) is ineffective in blocking hepatoma cell proliferation and antagonizes the inhibitory role of LAP on the cell cycle. Without being bound by theory, Buck, et al., *supra,* suggest that the 145 amino acids of LIP, including the leucine zipper and the basic domain, act as an antagonist of LAP by competing either directly for a DNA-binding site or indirectly by forming LIP/LAP dimers. Accordingly, the present invention provides recombinant vectors encoding the LAP (transciptional activator) activity wherein expression of LIP is inhibited. In another example, the present invention provides a recombinant vector encoding the LAP (transciptional activator) activity wherein expression of LIP is inhibited, and wherein expression of LIP is inhibited such that LIP does not down-regulate LAP activity. LAP activity can be measured by ability of the LAP to inhibit hepatoma cell proliferation in the assay disclosed in Buck, et al., *supra,* or by inhibiting cancer formation in the *in vivo* model disclosed herein in the examples.

Descombes et al., (1991, Cell, vol. 67:569-579) disclose that LAP mRNA has three in-frame AUGs. LAP is initiated at the first in-frame AUG (39 kd protein); and LIP is initiated at the third frame AUG (20kd protein). In some examples, the recombinant vector comprises the N-terminal LAP activation domain and/or the DNA-binding domain and/or the leucine zipper and expresses LAP activity wherein LIP activity is inhibited. The present invention provides recombinant vectors encoding LAP activity comprising nucleic acid encoding a variant LAP polypeptide wherein the initiating codon for LIP is substituted with another codon, wherein the substituted codon is not TTG, and, in some examples, is not a stop codon. The present invention also provides isolated nucleic acid encoding a variant LAP polypeptide wherein the initiating codon for LIP is substituted with another codon, wherein the substituted codon is not TTG, and, in some examples, is not a stop codon. The present invention also provides variant forms of LAP polypeptides wherein the initiating codon for LIP is substituted with another codon, wherein the codon is not TTG, and in some examples, is not a stop codon. As used herein, a "variant LAP polypeptide" is one that comprises a mutation of the initiating ATG codon of LIP such that LIP activity is reduced or suppressed as long as the variant LAP polypeptide retains at least one LAP biological activity.

The present invention provides recombinant gene vectors, in some examples, recombinant viral vectors, and in other examples, virus particles, comprising part or all of the C/EBP 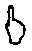 gene wherein the part or all of the C/EBP 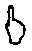 gene comprises the region around the initiation codon, ATG, of LIP wherein a mutation is introduced into a nucleotide sequence around the initiation codon, ATG, of LIP (transcription inhibitor protein). As used herein, the term "mutation" encompasses nucleic acid insertions, deletions, and substitutions. In some examples, the mutation is a substitute of the initiating codon, ATG of LIP with another codon. In some examples, the mutation decreases, reduces or suppresses transcription/translation of LIP. In some examples, the mutation decreases, reduces or suppresses transcription/translation of LIP without introducing a frameshift mutation in the nucleic acid encoding LAP. In other examples, the mutation decreases, reduces or suppresses transcription/translation of LIP while retaining a biological activity of LAP. In other examples, the recombinant vector comprises a nucleic acid fragment or portion of C/EBP 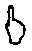 as long as that fragment or portion encodes a product that retains a LAP activity. LAP activity can be measured by methods known to one of skill in the art and by methods disclosed herein. In some examples, the initiating codon, ATG, of LIP is substituted with a codon encoding another amino acid, wherein the substituted codon is not TTG, and in some examples, wherein the substituted codon is not a stop codon. In some examples, the substituted codon is a conserved codon and is not TTG. In some examples, the codon encodes Ala, Gly or Pro. In other examples disclosed herein, the initiating codon ATG of LIP is substituted with CGC. Upon delivery of such a recombinant gene vector to a cell or tissue, the transcription/translation of LIP is decreased, reduced, stopped or suppressed. In other examples, the transcription/translation of LIP is decreased, reduced, stopped or suppressed without affecting the transcription/translation of LAP (transcription activator) from the C/EBP 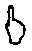 gene. In some examples, at least one biological activity of LAP is retained. In some examples, upon delivery of a recombinant vector of the present invention to a cell, LAP is predominantly expressed. Further, as demonstrated by the Examples of the present specification, when a vector comprising a C/EBP 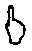 mutation introduced gene was injected IP into nude mice, the mice were found to have no cancer formation and metastasis as compared to controls. That is, in the present invention it has been revealed that cancer cells *in vivo* can be normalized by the predominant expression of only LAP.

The origin of a C/EBPβ (CCAAT/enhancer binding protein β) gene which can be used in the present invention is not particularly limited, and any gene derived from any living organism may be used. The present invention encompasses C/EBP 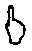 from any source. In some examples, a C/EBPβ gene obtainable from mammals may be used. The term "mammal" refers to any individual of a mammalian species, and includes large animals (cows, sheep, horses and the like), sport animals (including dogs and cats), and primates (including old world monkeys, new world monkeys, apes, humans, and the like). In other examples, human C/EBP 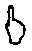 is used. In particular, the nucleotide sequence and amino acid sequence of human-derived C/EBPβ gene are described as SEQ ID NOS: 3 and 4, respectively.

The C/EBPβ (CCAAT/enhancer binding protein β) gene used in the present invention may be cDNA obtained from mammalian cultured cells or the like by using technologies known to a person skilled in the art, such as for example, PCR. Examples of mammalian cultured cell lines from which C/EBP 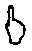 can be obtained include liver parenchymal cells, mammary epithelial cells and adipocytes. Such cell lines are available from public sources. Alternatively, a C/EBP 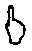 gene, or a fragment or portion thereof, e.g. a fragment or portion comprising the initiating codon ATG of LIP, may be a gene chemically synthesized on the basis of the information of the nucleotide sequences and amino acid sequences of SEQ ID NOS: 1 to 4 of the present specification.

Further, when a recombinant gene vector of the present invention comprising part or all of a C/EBP 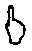 gene is used as an agent for gene therapy of humans, that is, for administration or delivery to humans, a human gene is preferably used in order to reduce, minimize or suppress any potential immunological rejection and to enhance therapeutic effects.

Methods for introducing mutation(s) into a nucleotide sequence around the initiation codon, ATG, of LIP in the nucleotide sequence of the C/EBPβ gene, are known to a person skilled in the art, and it is carried out by using common recombination techniques, such as by using PCR methods using properly designed primers. PCR technology is well known in the art.

The introduction of mutation(s) into a nucleotide sequence around the initiation codon, ATG, of the LIP (transcription inhibitor protein) is carried out or designed so as to decrease, reduce, stop or suppress the transcription/translation of LIP and in some examples, without affecting the transcription/translation of LAP (transcription activator). The phrase "without affecting the transcription/translation of LAP (transcription activator)" means that LAP having a biological activity is expressed without the introduced mutation creating the occurrence of a frameshift in the transcription/translation of LAP. LAP regulates transcription of various proteins, including FGF receptor and IL-8. LAP has been shown to arrest proliferation of HepG2 cells and decreases transcription from the *c-jun* promoter. See Buck et al, 1994, The EMBO J. vol 13: 851-860. One of skill in the art would be able to determine if a mutation introduced around the initiating codon, ATG, in LIP affects the biological activity of LAP by measuring for example, HepG2 proliferation or transcription from the *c-jun* promoter as disclosed in Buck et al. In addition, the phrase "to decrease, reduce, stop or suppress the transcription/translation of LIP" means that LIP having biological activity is expressed at a lower level as compared to a control or the LIP expression level is not detectable or LIP is not expressed from the mutant C//EBP 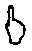 gene. Methods for assaying for transcription/translation of LAP and LIP are known to one of skill in the art and include Northern blot and PCR.

Further, the phrase "a nucleotide sequence around the initiation codon, ATG, of LIP" as used herein, encompasses a nucleotide sequence within the range of about 100 nucleotides forward (3') and backward (5') from the initiating ATG of LIP (corresponding to the 457^{th} and 459^{th} nucleotides in the nucleotide sequence of SEQ ID NO:1); and in some examples, about 90 nucleotides forward and backward from ATG; and in some examples, 80 nucleotides forward and backward from ATG, and in some examples, 70 nucleotides forward and backward from ATG 60 nucleotides forward and backward from ATG; and in some examples, 50 nucleotides forward and backward from ATG, and in some examples, 40 nucleotides forward and backward from ATG, in some examples, 30 nucleotides forward and backward from ATG; and in some examples 20 nucleotides forward and backward from ATG; in some examples, 10 or 5 nucleotides forward and backward from ATG; and in some examples, the ATG is substituted with a codon for another amino acid. In other examples, the ATG is left intact and nucleic acid around the ATG and in some examples, 3' to the ATG is mutated such that there is no transcription and/or translation from LIP.

As an example, the initiation codon, ATG, of LIP (transcription inhibitor protein) can be substituted with another codon, wherein the codon is not TTG and in some examples, is not a stop codon. The type of codon to be placed instead of ATG is not particularly limited, as far as the codon substitution is a codon other than a termination codon, and causes no frameshift for the translation of LAP. Buck et al., *supra*, disclose that the integrity of the LAP leucine zipper (found in the C-terminal 145 amino acids) is required to prevent hepatoma cells from entering S-phase. A codon encoding an amino acid that causes no influence on the properties of the encoded protein is preferable. Examples of such amino acids include alanine, glycine, and proline. In an illustrative embodiment disclosed herein, the initiating codon ATG of LIP is substituted with a codon encoding Arg. As shown in the examples, when a g6 breast cancer cell was transfected with a vector comprising this substitution, and introduced into nude mice, the nude mice lost cancer forming ability as compared to a control.

A recombinant gene vector of the present invention is a vector which comprises part or all of a C/EBPβ gene having the above described mutation introduced thereinto. The recombinant gene vector of the present invention is constructed by introducing, such as by ligating, a C/EBPβ gene having a nucleotide sequence having a mutation introduced thereinto to the downstream of a promoter in a suitable vector. Techniques for introducing mutations in gene sequences and constructing recombinant vectors are known by those of skill in the art.

In some examples, when the recombinant gene vector is introduced into a cell, it is also an expression vector capable of expressing part or all of LAP as a gene product in the cell. In some examples, the part or all of LAP retains at least one LAP activity. Epimorphin is known to increase expression of C/EBPβ (Hirai et al., 2001, J. of Cell Biol. Vol. 153 pg. 785-794). Therefore, in some examples of the present invention, nucleic acid encoding part or all of epimorphin is also introduced into a cell along with a recombinant vector of the present invention, in particular along with a recombinant vector that expresses part or all of the LAP gene product that has at least one LAP activity. Examples of these proteins or peptides having epimorphin activity include epimorphin per se (that is a full-length protein), peptides comprising a partial amino acid sequence of epimorphin and having epimorphin activity, and modified peptides thereof. Epimorphin and partial peptides thereof are disclosed in detail in EP Patent Publication No. 0698666, U.S. Pat. No. 5,726,298, U.S. Pat. No. 5,837,239, and International Patent Publication Nos. WO98/22505 and WO01/94382, and these epimorphin and partial peptides described therein can also be used. Nucleic acid encoding epimorphin is introduced into the cell before, after or simultaneous with a recombinant vector of the present invention and can on the same recombinant vector or on a different vector.

As described above, the present invention provides a C/EBPβ gene comprising a substitution of the initiation codon ATG of LIP (transcription inhibitor protein) substituted with another codon (as long as the codon is not TTG) incorporated into a recombinant vector of the present invention. The present invention encompasses methods for introducing a recombinant vector of the present invention into a host cell. In some embodiments, the vector is a recombinant viral vector and a host cell is infected with the viral vector. Therefore, the present invention encompasses a recombinant vector of the present invention that is characterized in that an introduction of the gene into a cell and expression of the gene in the cell are carried out by infecting the cell with the vector. Introduction of genetic constructs, such as for example, a recombinant vector of the present invention, into a cell can be accomplished using any technology known in the art, including calcium phosphate-mediated transfection, electroporation, lipid-mediated transfection, naked DNA incorporation, electrotransfer, and viral (both DNA virus and retrovirus mediated) transfection. Methods for accomplishing introduction of genes into cells are well known in the art.

### II. Viral Vectors

Expression plasmids for an animal, that is, a mammal, such as a human, can be used. In some examples, a vector is a virus vector. Examples of the vector to be used in the present invention include virus vectors such as retrovirus vectors, adenovirus vectors, adeno-associated virus vectors, baculovirus vectors, and vaccinia virus vectors. Among virus vectors, it is particularly desirable to use retrovirus vectors, because, after cells are infected with retrovirus vectors, the virus genome is incorporated into a host chromosome and the vector allows a foreign gene integrated into the vector to be expressed stably and over a long period. The construction of retrovirus recombinant vectors and their use *in vitro* or *in vivo* has been widely described in the literature: see in particular Breakfield et al., *New Biologist* 3 (1991) 203; EP 453242, EP 178220, Bernstein et al. Genet. Eng. 7 (1985) 235; McCormick, *BioTechnology 3* (1985) 689, and the like. The methodology of using replication-incompetent retroviruses for retroviral-mediated gene transfer of gene markers is well established (Correll, et al. (1989) *PNAS* USA 86:8912; Bordignon (1989), *PNAS* USA 86:6748-52; Culver, K. (1990), *PNAS* USA 88:3155; and Rill, D.R. (1991) *Blood* 79(10):2694-700.

Adenovirus has the advantage of effecting high efficiency of transduction and does not require cell proliferation for efficient transduction of cell. For general background references regarding adenovirus and development of adenoviral vector systems, see Graham et al. (1973) *Virology* 52:456-467; Takiff et al. (1981) *Lancet* 11:832-834; Berkner et al. (1983) *Nucleic Acid Research* 11: 6003-6020; Graham (1984) *EMBO J* 3:2917-2922; Bett et al. (1993) *J. Virology* 67:5911-5921; and Bett et al. (1994) *Proc. Natl. Acad. Sci. USA* 91:8802-8806. Adenoviral vectors have been used for the cloning and expression of genes in vitro (Gluzman et al., Cold Spring Harbor, N.Y. 11724, p. 187), for the creation of transgenic animals (WO95/22616), for the transfer of genes into cells ex vivo (WO95/14785; WO95/06120) or for the transfer of genes into cells in vivo (see in particular WO93/19191, WO94/24297, WO94/08026).

An adeno-associated virus (AAV) is a parvovirus which is a linear single stranded DNA virus of approximately 5kb that requires a helper virus (adenovirus, etc.) for virus replication. (see, B.J. Carter, in "Handbook of Parvoviruses", ed., P. Tijsser, CRC Press, pp.155-168). The use of vectors derived from AAVs for the transfer of genes *in vitro* and *in vivo* has been described in the literature (see in particular WO91/18088; WO93/09239; U.S. Pat. Nos. 4,797,368, 5,139,941, and EP 488 528). It is known that the adeno-associated virus is integrated into a specific site of the chromosome of a host cell through ITRs (inverted terminal repeat) that exist at both terminals of the viral genome and have a T-shaped hairpin structure. With respect to the virus protein, the left half of the genome encodes Rep of a nonstructural protein (regulatory protein), while the right half of the genome encodes Cap of a capsid protein, that is a structural protein. For the production of an AAV vector, an AAV is constructed that comprises ITRs at both terminals thereof, and a plasmid (AAV vector plasmid) having a gene of interest or nucleic acid comprising a gene of interest is inserted between ITRs. Virus protein necessary for the virus replication or the formation of virus particles is supplied from other helper plasmid. Both of the above plasmids are introduced into HEK293 cell, such as, for example, by transfection and then the resultant cell is infected with adenovirus (helper virus), thereby producing a nonproliferating recombinant AAV (AAV vector). Alternatively, the host cell comprises nucleic acid encoding helper virus function. This AAV vector exists within nucleus and therefore after freeze-thawing and collecting cells, contaminating adenoviruses are inactivated by heating or are removed from the AAV, e.g., by CsCl gradient. Further, vectors are purified by the cesium chloride density-gradient ultracentrifugation method.

Baculovirus has been used for expressing proteins in mammalian cells, see U.S. Pat. No. 5,731,182, for example. The genome of a baculovirus may be modified by insertion of ligand DNA, which comprises a gene encoding a mammalian receptor specific protein that allows the baculovirus to bind and enter the mammalian cell.

Vaccinia viruses are described in US patent no. 6,103,244. Construction of recombinant vaccinia viruses comprising foreign genes has been described by Panicali and Paoletti, (1982, *Proc. Nat'l Acad. Sci.* U.S.A. 79:4927-4931; Mackett et al., (1982, *Proc. Nat'l Acad. Sci.* U.S.A. 79:7415-7419; and U.S. Pat. No. 4,769,330.

When retrovirus vectors are employed, examples thereof include those derived from an oncovirus such as moloney murine leukemia virus (MoMLV) and those derived from lentivirus such as human immunodeficiency virus (HIV).

A commonly used retrovirus vector is one utilizing a fundamental structure of the murine leukemia virus (MoMLV) which is an RNA virus, and it has a broad host range and relatively high efficiency of gene introduction. Methods for preparing retrovirus vectors are known in the art. Briefly, for the preparation of retrovirus vectors, firstly a large portion of gag, pol, and env among LTR (long terminal repeat) is deleted from the virus genome, and instead of them, a gene of interest is inserted. When this vector plasmid is introduced into a packaging cell line prepared for expressing the gene product, virus proteins (gag, pol, and env), that is a cell line comprising nucleic acid for gag, pol, and env, recombinant retroviruses expressing the gene product (retrovirus vectors) are produced in the culture supernatant. Usually, a high-titer retrovirus vector producing line is cloned, and the cell line is used over a long period. Target cells are generally infected using the culture supernatant of the above-described virus vector producing cells.

An adenovirus is a linear double stranded DNA virus having a size of approximately 36kb. Methods for preparing adenovirus vectors are known in the art and are described briefly below. In some examples, a replication-deficient adenovirus, such as an adenovirus lacking part or all of the essential E1 function, is used. A preparation method of adenovirus vectors is briefly described below. Briefly, an E1 gene region is deleted from the adenovirus, and a cosmid having a gene of interest inserted into that region is constructed. This cosmid is introduced into HEK293 cell together with a parent virus DNA (one having a terminal protein attached thereto is used) in which an E1 gene region has been excised. Then, homologous recombination occurs in the cell, resulting in the production of nonproliferating adenovirus vectors. These virus vectors are collected by freeze thawing of the cells and purified by the cesium chloride density-gradient ultracentrifugation method. The features of adenovirus vectors are that the vector can produce high-titer vectors, can introduce a gene efficiently into a broad range of cells, and can introduce a gene into nondividing cells. When the gene is introduced into cancer cells, a little cytotoxicity does not matter. See Horowitz J. 1999, *Curr. Opin. Mol. Ther.* 4:500-509. Further, there are some examples wherein transient gene expression can attain therapeutic effects, and thus adenovirus vectors are particularly suitable for gene therapy for cancers.

Since animal cells are used as hosts, promoters such as promoters derived from SV40, retrovirus promoters, metallothionein promoters or β actin promoters, can be used. Further, enhancers may be used if necessary. Cell or tissue specific expression can be achieved by using cell-specific enhancers and/or promoters. See generally Huber et al. (1995) *Adv. Drug Delivery Reviews* 17:279-292. Expression vectors comprising a recombinant vector of the present invention may also comprise one or more promoter(s) and/or enhancer(s) of tumor-marker proteins or other factors upregulated in tumors which can target the vector to the tumor cell. For example, promoters of ErbB2, the augmented expression of which is found in mammary tumor cells, could be utilized to target a recombinant vector of the present invention to mammary tumor cells.

As a host of a plasmid vector for the expression in animals, *Escherichia coli* K12·HB101 strain, DH5α strain or the like can be used. Such strains are available from public sources. Methods for transforming *E. coli* are known to a person skilled in the art. (See for example, Maniatis et al.). As a host for a virus vector, animal cells having the ability to produce virus, such as COS-7 cells, CHO cells, BALB/3T3 cells and HeLa cells are used. Virus vectors may be replication-competent or replication-deficient. Replication-deficient virus vectors are grown in appropriate helper cell lines, i.e., cell lines comprising nucleic acid encoding the virus functions essential for replication. As a host for a retrovirus vector, ΨCRE, ΨCRIP, MLV, or the like may be used. As a host for an adenovirus vector and an adeno-associated virus vector, HEK293 cells derived from the human embryonic kidney or the like may be used. The introduction of virus vectors into animal cells can be carried out by a calcium phosphate method, or other methods known to those of skill in the art.

The obtained transformants are cultured as follows to produce recombinant gene vectors.

The cultivation of *E. coli* transformants can be carried out using a liquid medium with pH level of 5 to 8, which contains a carbon source, nitrogen source, inorganic substance and others necessary for the growth thereof. Cultivation is usually conducted at 15 to 43C° for approximately 8 to 24 hours. After cultivation, recombinant gene vectors of the present invention can be obtained by a conventional DNA isolation and purification method.

The cultivation of animal cell transformants can be carried out using a medium, such as 199 medium, MEM medium, and DMEM medium, which contains approximately 5 to 20% fetal bovine serum. Preferable pH value of the medium is from approximately 6 to 8. The cultivation is usually conducted at approximately 30 to 40C° for approximately 18 to 60 hours. Virus particles containing the recombinant gene vectors of the present invention are released into the culture supernatant. The condensation and purification of virus particles is carried out by methods known to a person skilled in the art, such as the cesium chloride centrifugation method, the polyethyleneglycol precipitation method, and the filter concentration method, thereby obtaining the recombinant gene vectors of the present invention.

### III. Control of LIP

The present invention provides compositions and methods for decreasing, reducing or suppressing the transcription and/or translation of LIP in a host cell. Such compositions include antisense nucleic acid, oligonucleotides, that is oligonucleotide decoys, ribozymes, and interfering RNAs (iRNAs) capable of decreasing, reducing or suppressing the transcription of LIP in a host cell. Accordingly, the present invention provide methods for decreasing or suppressing the transcription and/or translation of LIP in a cell comprising contacting the cell with an antisense nucleic acid, an oligonucleotide, a ribozyme, and/or interfering RNAs (iRNAs) that is capable of decreasing, reducing or suppressing the transcription of LIP in the cell. The transcription and translation of LIP can be measured by means known to one of skill in the art and include PCR and Northern blot.

The present invention provides oligonucleotides of about 10 to about 100 nucleotides in length comprising a nucleotide sequence around the initiation codon, ATG, of LIP (transcription inhibitor protein) in the nucleotide sequence of a C/EBPβ (CCAAT/enhancer binding protein β) gene, or a complementary sequence thereof. In some examples, the oligonucleotide is capable of decreasing, reducing, or suppressing LIP function in a cell.

In some examples, an oligonucleotide has a length of about 10 to about 80 nucleotides, in other examples, an oligonucleotide has a length of about 15 to about 50 nucleotides; in other examples, an oligonucleotide has a length of about 20 to about 80 nucleotides; in other examples, an oligonucleotide has a length of about 15 to about 40 nucleotides; in other examples, an oligonucleotide has a length of about 15 to about 30 nucleotides; in other examples, an oligonucleotide has a length of about 15 to about 25 nucleotides. In other examples, an oligonucleotide is at least about 10, 11, 12, 13, 14, 15, 16, 17, 18, 19, or at least about 20 nucleotides in length. In other examples, an oligonucleotide is up to about 30, 40, 50, 60, 70, or 80 nucleotides in length. An oligonucleotide comprising a nucleotide sequence around the initiation codon, ATG, of the LIP (transcription inhibitor protein) is used as a decoy oligonucleotide to decrease, reduce or suppress LIP expression. Further, the oligonucleotide having a sequence complementary to the above nucleotide sequence is used as an antisense oligonucleotide to decrease, reduce or suppress LIP expression. For example, a nucleic acid comprising the sequence which is from the 427^{th} nucleic acid G to the 489^{th} nucleic acid C of SEQ ID NO:1 (63 bases), or the complementary strand thereof, and a nucleic acid comprising the sequence which is from the 565^{th} nucleic acid G to the 627^{th} nucleic acid T of SEQ ID NO:3 (63 bases), or the complementary strand thereof, are regions from which oligonucleotide decoys and/or antisense nucleic acid can be designed. An oligonucleotide (DNA or RNA), that is an oligonucleotide decoy or antisense nucleic acid, is inserted in a vector and transfected into a cell in order to decrease or reduce the expression level of LIP in the cell. Without being bound by theory, the promoter or the like of LIP binds to the oligonucleotide. An oligonucleotide of the present invention can be used alone or together with a recombinant vector of the present invention or together with other treatments.

In both cases described above, the expression of LIP is decreased, reduced or suppressed, and as a result the ratio of the expression level of LAP (transcription activator) to that of LIP (transcription inhibitor protein), both being expressed from the C/EBPβ (CCAAT/enhancer binding protein β) gene, increases. As demonstrated herein in the Examples, in an *in vivo* model of cancer, decreasing the expression of LIP, thereby increasing the ratio of the expression of LAP to LIP, had the result of reducing cancer formation and metastasis as compared to a control. Therefore, the administration or introduction to a cell of an oligonucleotide or antisense nucleic acid or iRNA that is capable of decreasing, reducing or suppressing LIP expression is correlated with a reduction in the symptoms associated with cancer including for example, cancer formation and/or metastasis and slowing of tumor growth for example.

Interfering RNA (iRNA) is the mechanism of sequence-specific, post-transcriptional gene silencing initiated by double-stranded RNAs (dsRNA) homologous to the gene being suppressed. See Sharp, P. (2001, Genes & Development, 15:485-490). A dsRNA comprised of a mRNA plus its complementary strand form an RNA-RNA duplex. This duplex region is degraded by an RNAse III like enzyme and the mRNA cannot be translated. When introduced into a cell, a short dsRNA(small interfering RNA or siRNA) is also efficient in degrading mRNAs containing the sequence of the short dsRNA. Without being bound by theory, evidence suggests a cellular RNA degradation system is present in cells, likely to be stimulated by dsRNA as a means to protect against invasion by viral or transposon RNAs. The present invention encompasses interfering RNA sequences that comprise a nucleotide sequence around the initiating codon, ATG of LIP and which are capable of decreasing, reducing, and/or suppressing LIP expression. In some examples, the iRNA is designed to target the initiating ATG of LIP, which corresponds to the 457th to 459th nucleotides in the nucleotide sequence of SEQ ID NO: 1, and to the 595th to 597th nucleotides in the nucleotide sequence of SEQ ID NO: 3. Means for designing iRNAs are known in the art. An iRNA of the present invention can be used alone or together with a recombinant vector of the present invention and/or together with a mutant LAP polypeptide that has LAP activity and/or together with other treatments.

Alternatively, the present invention encompasses the use of ribozymes that would decrease, reduce, stop or suppress LIP transcription and in some examples, without effecting LAP expression. A ribozyme that is capable of decreasing, reducing or suppressing LIP can be used alone or together with a recombinant vector of the present invention and/or together with a mutant LAP polypeptide that has LAP activity and/or together with other treatments.

Assays for screening for particular oligonucleotides, antisense nucleic acid and iRNAs that are capable of decreasing, reducing and/or suppressing LIP expression are known in the art and described herein. Briefly, in the assay disclosed herein in the examples, the g6 breast cancer cell line is contacted with a test sample(s), for example, a recombinant vector, an oligonucleotide, antisense or iRNA of the present invention, and are examined histologically, for morphology and cell adhesion. In a cell contacted with a recombinant vector, an oligonucleotide, antisense or iRNA capable of decreasing, reducing and/or suppressing LIP expression, the morphology will be similar to normal, non-cancerous cells. Also, such cells can be introduced into nude mice and cancer forming ability and metastasis can be assayed. A recombinant vector, such as a viral vector comprising a nucleotide sequence for part or all of the CCAAT/enhancer binding protein β (C/EBPβ) gene wherein said part or all of the C/EBPβ gene comprises nucleic acid around the initiation codon, ATG, of the LIP transcription inhibitor protein, and wherein the nucleotide sequence comprises a mutation in the ATG of said LIP transcription inhibitor protein can be used as a control. The recombinant vector described herein in the examples can be used as a control in screening assays.

### IV. Compositions and Uses

### Compositions

The present invention encompasses compositions comprising the recombinant vectors, recombinant viral vectors or viral particles, oligonucleotides, antisense RNA or iRNA of the present invention. In some examples, a composition may further comprise a pharmaceutically acceptable excipient or carrier, or a buffer.

In some examples, such compositions are used in methods for the treatment and/or amelioration of the symptoms of a disease or condition associated with the ratio of LAP to LIP in a cell or individual, such as cancer and/or tumor growth. In some examples, the compositions disclosed herein exhibit oncolytic activity. As used herein, the term "treating" or "treatment" refers to ameliorating, improving, reducing, or stabilizing one or more symptoms of a disease or undesired condition, such as cancer, as well as slowing progression of one or more symptoms of the disease or undesired condition. A composition of the present invention may or may not be used in conjunction with other treatment modalities, including but not limited to chemotherapeutic agents known in the art, radiation and/or antibodies. In some examples, a composition of the present invention is administered in combination with part or all of epimorphin.

As used herein, the terms "malignant", "malignant cells", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation. The term "tumors" includes metastatic as well as non-metastatic tumors.

As used herein "oncolytic activity" refers to inhibition or suppression of tumor and/or malignant and/or cancerous cell growth; regression of tumor and/or malignant and/or cancerous cell growth; cell death of tumor and/or malignant and/or cancerous cells or prevention of the occurrence of additional tumor and/or malignant and/or cancerous cells. As used herein, "inhibiting or suppressing tumor growth" refers to reducing the rate of growth of a tumor, halting tumor growth completely, causing a regression in the size of an existing tumor, eradicating an existing tumor and/or preventing the occurrence of additional tumors upon administration of the VSV comprising compositions, or methods of the present invention. "Suppressing" tumor growth indicates a growth state that is curtailed when compared to growth without contact with a composition of the present invention. Tumor cell growth can be assessed by any means known in the art, including, but not limited to, measuring tumor size, determining whether tumor cells are proliferating using a ³H-thymidine incorporation assay, or counting tumor cells. "Suppressing" tumor and/or malignant and/or cancerous cell growth means any or all of the following states: slowing, delaying, and stopping tumor growth, as well as tumor shrinkage. "Delaying development" of tumor and/or malignant and/or cancerous cells means to defer, hinder, slow, retard, stabilize, and/or postpone development of the disease. This delay can be of varying lengths of time, depending on the history of the disease and/or individual being treated.

The present invention encompasses compositions and methods of increasing the ratio of LAP to LIP using a recombinant vector of the present invention or an oligonucleotide, antisense nucleic acid or iRNA that is capable of decreasing, reducing and/or suppressing LIP expression, in particular LIP expression as associated with malignant cells and/or tumor cells as described herein. Individuals indicated for treatment are individuals who are considered to be at risk for developing cancer, tumor or malignant cells, such as those who have had previous disease comprising cancer, malignant cells or tumor cells or those who have had a family history of such cancer, tumor cells or malignant cells. Determination of suitability of administering a composition of the invention will depend on assessable clinical parameters such as serological indications and histological examination of cell, tissue or tumor biopsies. Generally, a composition in a pharmaceutically acceptable excipient is administered.

Accordingly, the present invention provides methods for suppressing cancer or tumor growth, comprising the step of contacting the cancer or tumor cell with a recombinant vector or mutant LAP polypeptide, or nucleic acid construct, such as an oligonucleotide or antisense nucleic acid or iRNA, of the present invention, thereby increasing the LAP to LIP ratio in the tumor or cell.

In other examples, such compositions are used in methods for screening for agents that modulate the ratio of LAP to LIP in a host cell. In some examples, screening methods are used to identify agents that decrease, reduce or suppress expression of LIP in a host cell. In other examples, screening methods are used to identify agents that increase the expression of LAP in a host cell or increase the biological activity of LAP in a host cell. In other examples, an agent or a recombinant vector or nucleic acid construct has oncolytic activity as measured in screening assays disclosed herein.

In yet other examples, the present invention provides methods for determining whether a cancer cell, malignant cell or tumor cell is susceptible to treatment with a composition of the present invention, that comprise (a) dividing a sample containing cells of the cancer, malignant or tumor into a first portion and a second portion; (b) treating the first portion with the composition, such as a viral vector or a recombinant vector comprising a nucleotide sequence for part or all of the CCAAT/enhancer binding protein β (C/EBPβ) gene wherein said part or all of the C/EBPβ gene comprises a region around the initiation codon, ATG, of the LIP transcription inhibitor protein, and wherein the nucleotide sequence comprises a mutation in the initiation codon, ATG, of said LIP transcription inhibitor protein or an oligonucleotide or antisense nucleic acid or iRNA that is capable of reducing LIP transcription or an agent identified by the screening methods disclosed herein; and (c) determining whether the percentage of dead cells in the first portion is higher than in the second portion, wherein the cancer, malignant or tumor is susceptible to treatment with the composition if the percentage of dead cells in the first portion is higher than in the second portion.

In some examples, the present invention further relates to an agent for gene therapy, that is, to an agent which is administered or delivered to a cell, wherein the agent comprises a recombinant gene vector comprising a nucleotide sequence for part or all of the C/EBP 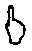 gene wherein an initiation codon, ATG, of LIP (transcription inhibitor protein) in the nucleotide sequence of a C/EBPβ (CCAAT/enhancer binding protein β) gene is substituted with another codon, wherein the substituted codon is not TTG, or which comprises an oligonucleotide or antisense nucleic acid or iRNA of the present invention. Such a composition can be used in methods for the treatment and/or amelioration of the symptoms of cancer, such as for example, suppression or slowing of tumor growth. In some examples, such an agent will have oncolytic activity when administered locally to the tumor cells or malignant cells, that is intratumorally, as well as when administered distal to the tumor or malignant cell, such as *via* intravenous administration or by other routes. In some examples, the administration of a recombinant vector or mutant LAP polypeptide, or nucleic acid construct, such as an oligonucleotide or antisense nucleic acid or iRNA, of the present invention, is performed *ex vivo* with the treated cells being returned to the individual after treatment.

Examples of cancers to be treated include, but are not limited to, malignant melanomas, malignant lymphoma, digestive organ cancers, lung cancers, esophagus cancers, gastric cancers, large intestine cancers, rectum cancers, colon cancers, ureteral tumors, gallbladder cancers, bile duct cancers, biliary tract cancers, breast cancers, liver cancers, pancreas cancers, testicular tumors, maxillary cancers, lingual cancers, lip cancers, oral cavity cancers, pharyngeal cancers, laryngeal cancers, ovarian cancers, uterine cancers, prostatic cancers, thyroid cancers, brain tumors, Kaposi's sarcoma, hemangioma, leukemia, polycythemia vera, neuroblastomas, retinoblastomas, myelomas, bladder tumors, sarcomas, osteosarcomas, muscle tumors, skin cancers, basal cell cancers, skin appendage carcinomas, metastasized skin carcinomas, and skin melanomas. Preferable examples of cancers to be treated include breast cancers and liver cancers.

The composition for use in methods of the present invention of the present invention can be prepared by blending recombinant gene vectors, such as virus vectors, (or oligonucleotide or antisense nucleic acid or iRNA) as active components with base materials.

Further, when the recombinant vector is integrated into a virus vector, a composition for gene therapy, that is, an agent for delivery to a cell can be produced by preparing virus particles comprising recombinant DNAs and blending them with the base materials.

Any base materials commonly used for injections may be employed as the base materials to be used for the agent for gene therapy, and examples thereof include distilled water, salt solutions such as sodium chloride or mixtures of sodium chloride and inorganic salts, solutions of mannitol, lactose, dextran, glucose or the like, amino acid solutions of glycine, arginine or the like, mixture solutions of glucose solution with organic acid solutions or salt solutions. Alternatively, in addition to these base materials, according to conventional methods known to a person skilled in the art, injections can be prepared as solutions, suspensions, or dispersions by using adjuvants such as osmoregulators, pH regulators, vegetable oil or surfactants. These injections can be also prepared by operations such as powderization and freeze-drying as formulations to be dissolved before use. Further, the agent of the present invention is encapsulated in liposome or others, if necessary, just before the administration, and thus it can be used for treatment and/or amelioration of the symptoms for cancers.

As methods for introducing a composition of the present invention into living organisms, the following methods are known: methods for chemical or physical introduction of genes (transfection); and methods using viruses (transduction).

Examples of the methods for physically introducing a gene into living organisms (transfection) include *in vivo* electroporation method and the gene gun method. *In vivo* electroporation is a method for introducing DNAs into living tissues by applying voltage pulse directly to the living tissues. DNAs are dissolved into a suitable buffer solution (e.g. 1mM Tris, 25µM EDTA, 150mM NaCl) and the resultant solution is injected into tissues using a glass electrode. In the gene gun method, DNAs attached to gold particles is accelerated and introduced into cells. Under atmospheric pressure, a handheld type Helios gun developed by Biorad enables genes to be directly introduced into individuals in a simple manner at a high efficiency rate by an *in vivo* method. See Kuo C. F. et al. 2002, *Methods of Mol. Med.* 69:137-147. The gene gun method has the following advantages that there is no effect by DNA decomposition systems such as endosomes, gene can be introduced into any type of tissue, and gene can be introduced into specific site.

Examples of methods for chemically introducing a gene into a living organism include the liposome method, the membrane fusion protein-liposome method, and the lipofection method. Nidome T and Huang L, 2002, *Gene Ther.* 24: 1647-1652.

A liposome is a vesicle formed by polar lipids such as phospholipids in an aqueous phase. In forming a liposome, genes incorporated into the liposome are held in the membrane. Further, it is possible to carry out missile therapy wherein a specific protein is chemically bound to a liposome and the resultant product is concentrated on a cell of interest, that is, to target delivery to a cell of interest.

In the membrane fusion protein-liposome method, a virus envelope which is an entry means of various viruses into a cell, is bound to a liposome. For example, a membrane fusion protein of the Sendai virus, which has high fusion ability under neutral conditions and produces a multinucleated cell by cell fusion, can be used.

The lipofection method is a method using cationic lipids. It is considered that cationic lipids neutralize the negative charge of introduced genes, and the cationic lipids will simultaneously neutralize the negative charge of the plasma membrane surface, and fuse with the membrane due to the hydrophobicity of the lipid, thereby entangling the DNAs. Examples of specific commercial products of the cationic lipids include lipofectin, lipofectamine, and transfectam. These cationic lipids are considered to have liposome-like structures and the introduced genes are bound to the liposome surface.

Transduction is a method for introducing a gene into a living organism using viruses, which enables genes to be introduced at a high efficiency rate. Specifically, retrovirus vectors, adenovirus vectors, adeno-associated virus vectors or the like can be used.

Pharmaceutically acceptable carriers are well known in the art and include but are not limited to saline, buffered saline, dextrose, water, glycerol, sterile isotonic aqueous buffer, and combinations thereof. One example of such an acceptable carrier is a physiologically balanced culture medium containing one or more stabilizing agents such as stabilized, hydrolyzed proteins, lactose, etc. The carrier is preferably sterile. The formulation should suit the mode of administration.

The composition, if desired, can also contain minor amounts of wetting or emulsifying agents, or pH buffering agents. The composition can be a liquid solution, suspension, emulsion, tablet, pill, capsule, sustained release formulation, or powder. Oral formulation can include standard carriers such as pharmaceutical grades of mannitol, lactose, starch, magnesium stearate, sodium saccharine, cellulose, magnesium carbonate, etc.

Generally, the ingredients are supplied either separately or mixed together in unit dosage form, for example, as a dry lyophilized powder or water free concentrate in a hermetically sealed container such as an ampoule or sachette indicating the quantity of active agent. Where the composition is administered by injection, an ampoule of sterile diluent can be provided so that the ingredients may be mixed prior to administration.

The precise dose of a composition or an agent within the composition to be employed in the formulation will also depend on the route of administration, and the nature of the cell or individual, such as a human, being treated and should be decided according to the judgment of the practitioner and the circumstances according to standard clinical techniques. The exact amount of an agent utilized in a given preparation is not critical, provided that the minimum amount of the composition necessary to produce desired activity, e.g, oncolytic activity is given. A dosage range of as little as about 10 mg, up to amount a milligram or more, is contemplated.

Effective doses of a vector or viral particle or nucleic acid composition of the invention may also be extrapolated from dose-response curves derived from animal model test systems.

A dose of the agent for gene therapy of the present invention varies depending on the age, the gender, or the condition of a patient, or the route or the times of administration, or the dosage form of agent. In general, a dose of the recombinant vector for the adult per day is within the range from about 1 µg/kg to 1000 mg/kg, an in other examples, about 10 µg/kg to 100 mg/kg. The time of administration is not particularly limited. If administered as a virus, from about 10² up to about 10⁷ p.f.u., in other examples, from about 10³ up to about 10⁶ p.f.u., and in other examples, from about 10⁴ up to about 10⁵ p.f.u. can be administered. If administered as a polynucleotide construct, for example, a recombinant vector (i.e., not packaged as a virus), about 0.01 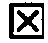 g to about 100 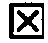 g of a construct of the present invention can be administered, in other examples, 0.1 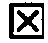 g to about 500 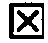 g, and in other examples, about 0.5 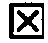 g to about 200 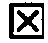 g can be administered. More than one composition can be administered, either simultaneously or sequentially. In some examples, a composition of the present invention is administered in combination with part or all of epimorphin. Administrations are typically given periodically, while monitoring any response. Administration can be given, for example, intratumorally, intravenously or intraperitoneally.

Many methods may be used to administer or introduce the compositions of the present invention, such as virus vectors or virus particles into individuals, including but not limited to, oral, intradermal, intramuscular, intraperitoneal, intravenous, intratumor, subcutaneous, and intranasal routes. The individual to which a composition is administered is a primate, or in other examples, a mammal, or in other examples, a human, but can also be a non-human mammal including but not limited to cows, horses, sheep, pigs, cats, dogs, hamsters, mice and rats. As the administration forms of the agent of the present invention, conventional systemic administration such as intravenous or intraarterial administration may be employed, or local administration such as local injections or oral administration against a carcinogenic lesion or potential metastasis site may be employed. Further, for the administration of the agent of the present invention, administration forms combined with catheter techniques, gene introduction techniques, surgical techniques or the like may be also employed.

Moreover, epimorphin is known to have a function of increasing the expression of C/EBPβ (Hirai et al., Journal of Cell Biology, Vol.153, No.4, 2001, 785-794). Therefore, the agent for gene therapy of the present invention can further enhance its therapeutic effects by using it in combination with a protein or peptide having epimorphin activity.

### Uses

### Modifying the ratio of expression level of LAP (transcription activator)/LIP (transcription inhibitor protein)

By using a composition of the present invention, such as a pharmaceutical composition comprising a recombinant gene vector described herein or an oligonucleotide or an antisense nucleic acid or an iRNA capable of decreasing, reducing or suppression LIP expression or capable of increasing LAP expression, the ratio of the expression level of LAP (transcription activator) activity to LIP (transcription inhibitor protein) activity, being expressed in the cell, can be changed. Accordingly, the present invention provides methods for modifying the ratio of the expression level of LAP (transcription activator) to LIP (transcription inhibitor protein) in a cell, comprising contacting the cell with a recombinant vector described herein or an oligonucleotide or antisense nucleic acid or iRNA described herein under suitable conditions. This method is also included in the scope of the present invention. In particular, according to the present invention, the anticancer activity, or oncolytic activity, or for example, the tumor suppression activity of a composition of the present invention can be accomplished by changing the ratio of the expression level of LAP (transcription activator) to LIP (transcription inhibitor protein) so as to increase the ratio (LAP expression/LIP expression) of LAP (transcription activator) to LIP (transcription inhibitor protein). Namely, a method for treating and/or ameliorating the symptoms associated with cancer, such as for example, suppressing tumor growth, which comprises a step of setting back an abnormal ratio of the expression level of LAP (transcription activator) / LIP (transcription inhibitor protein) in a cancer patient to a normal ratio, is included in the scope of the present invention.

### Method for screening anticancer agents

The present invention further relates to a method for screening an agent using the ratio of the expression level of LAP (transcription activator) and LIP (transcription inhibitor protein), both being expressed from C/EBPβ (CCAAT/enhancer binding protein β) gene, as an index. By the screening method of the present invention, a substance that can set back an abnormal ratio of the expression level of LAP (transcription activator) / LIP (transcription inhibitor protein) in a cancer patient to a normal ratio, can be selected. The thus selected substance that can set back an abnormal ratio of the expression level of LAP (transcription activator) / LIP (transcription inhibitor protein) in a cancer patient to a normal ratio is useful to treat and/or ameliorate the symptoms of cancer, such as for example, slowing the growth of tumors.

The ratio of the expression level of LAP (transcription activator) and LIP (transcription inhibitor protein), both being expressed from C/EBPβ (CCAAT/enhancer binding protein β) gene, can be determined by conventional methods known to a person skilled in the art, such as Northern blotting method, RT-PCR method, or Western blotting method. Probes, primers or antibodies to be used for the detection of LAP and LIP in these methods, can be appropriately obtained or prepared by a person skilled in the art in accordance with conventional methods using the nucleotide sequences and amino acid sequences of LAP and LIP described in the present specification.

The type of test substances which are subjected to the screening method of the present invention is not particularly limited, and any substance can be used. The test substance may be an oligonucleotide, an antisense nucleic acid, an iRNA, a low molecular weight synthetic compound or a compound existing in an extract from a naturally occurring substance, or it may be a compound library, phage display library, or a combinatorial library. In some examples, the test substance is a low molecular weight compound, and a compound library for low molecular weight compounds is preferable. The construction of a compound library is known to a person skilled in the art, and commercially available compound libraries may be also used.

The present invention will be described in detail in the following examples, but the scope of the present invention is not limited by these examples.

### Examples

### Example 1: Construction of vector

A vector (Hirai et al J.Cell Biol, Vol.153, No.4, 2001, 785-794) obtained by inserting the full sequence of rat LAP into pTetT-splice (GIBCO BRL) was used as a template, and the following primers (primer 1: GGG GGA TCC CGC CAT GGA AGT GGC CAA CTT CTA CTAC (SEQ ID NO: 5); and primer 2: ATA TGC TAG CGC GGG CGC GTC GTC CGC GCG CTT GCA (SEQ ID NO:6)) and LATag (TAKARA) were used to construct LAP cDNA with the deletion of the LIP region, followed by treatment with *Bam* HI and *Nhe* I, so that the terminal thereof became a restriction site.

A vector of Promega pTARGET (PROMEGA) was treated with *Nhe* I and *Bam* HI, and the cDNA prepared in the above (a) was inserted into the this vector using a ligation kit (TAKARA).

PtetLAP was used as a template, and the following primers (primer 3: ATA TGC TAG CGG CCG GCT TCC CGT TCG CCC TGC GCG (SEQ ID NO:7); and primer 4: ATA TGC TAG CAG TGA CCC GCC GAG GCC AGC AGC GGC (SEQ ID NO:8)) and LATag (TAKARA) were used to construct LIP region cDNA, followed by treatment with *Nhe* I, so that the terminal thereof became an *Nhe* I site.

The plasmid of the above (b) which were previously proliferated in *E. coli* and purified, were cleaved with *Nhe* I, and the terminals were dephosphorylated with alkaline phosphatase (TAKARA BAP). The DNA of the above (c) was inserted thereinto using a Takara ligation kit.

By determining the sequence, it was confirmed that LIP translation initiation codon, ATG, within the plasmid LAP was substituted with CGC (confirmation of mutation-introduced LAP).

After *Eco* RI site of pTet-splice vector (GIBCO BRL) was cut and treated with BAP, the *Eco* RI cut fragments comprising the mutation-introduced LAP that was excised from the DNA of the above (e) were inserted thereinto (construction of pTet-splice mutation-introduced LAP).

### Example 2: Cell culture and gene introduction

g6 cells (breast cancer cell line) (Desprez et al., 1993, Mol. Cell Differ. 1:99-110: Roskelley et al. 1994, Proc. Natl. Acad. Sci. USA. 91, 12378-12382; Hirai et al.,1998, J.Cell.Biol. 140:159-169) were maintained in a growth medium (DME/F12 [GIBCO BRL] added with 5% FBS [Hyclone], 5 µg/ml insulin [Sigma-Aldrich], and 50 µg/ml gentamicin). g6 cells (5×10⁵) were transfected with the vectors (5 µg) obtained in Example 1, pTet.tTAK vectors (Life Technologies) (5 µg), and pSV40neo (Schmidhauser et al., 1992, *Mol. Biol. Cell.* 3:699-709) (0.5 µg) using lipofectamine (Life Technologies) in accordance with the manual provided by the manufacturer. In the continuous presence of tetracycline, neomycin-resistant clones were selected, and thereafter the expression of LAP in the presence or absence of 5 µg/ml tetracycline was analyzed by Western blotting. g6LAP, g6LAP', and g6LAP" cell lines were isolated by this method.

### Example 3: Morphological changes of cells by mutation-introduced LAP

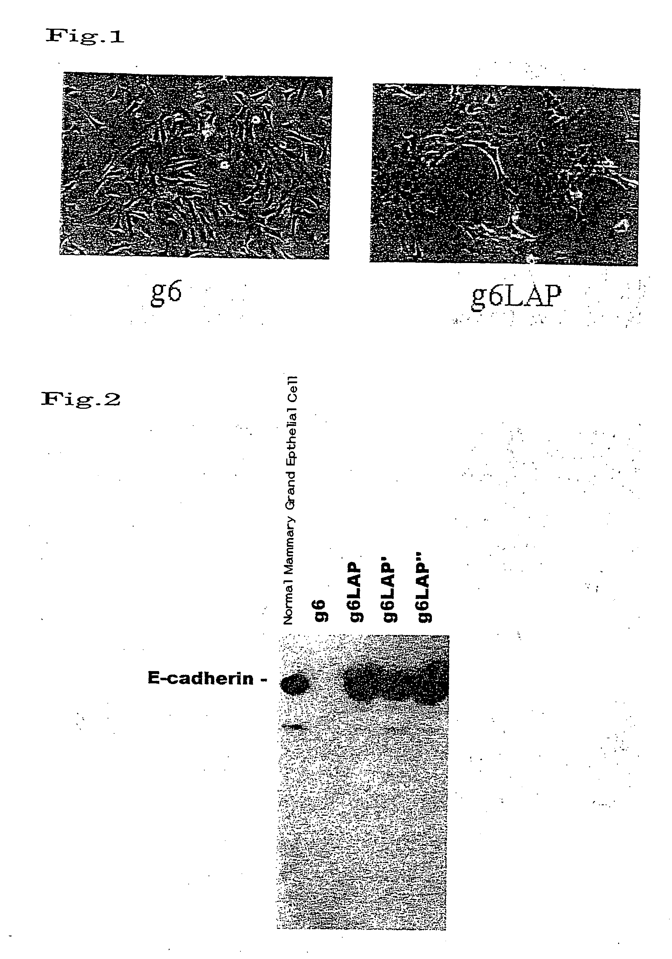

The morphologies of a g6 cell and a g6 LAP cell are shown in Fig. 1. When being cultured in the absence of tetracycline, it was observed that cells with the expression of mutation-introduced LAP genes caused cell adhesion and the cells were morphologically similar to normal cells.

### Example 4: Induction of expression of E-cadherin by mutation-introduced LAP

Western blotting was carried out by a conventional method. 500 µl of SDS sample buffer was added to cells cultured in a 24-well plate. The cells were collected and sonicated. The obtained samples were subjected to 4 to 20% gel electrophoresis and blotted on a PVDF membrane, followed by 1-hour blocking with TBS containing 5% skim milk (TBST), and then were reacted with anti E-cadherin antibodies (ECCD2, TAKARA) diluted 500-times in TBST for 1 hour. 10-minute washing with TBS was repeated twice. The resultants were reacted with anti rat Ig HRP labels (Amersham Pharmacia) diluted 1000 times in TBST for 1 hour. The resultants were sufficiently washed 3 times each for 10 minutes with TBS. Then, autoradiography was conducted using ECL (Amersham Pharmacia). The results are shown in Fig. 2.

As is understood from the results of Fig. 2, the expression of E-cadherin was induced as a result of inducing the expression of mutation-introduced LAP (3 different clones, g6LAP, g6LAP', and g6LAP") in a medium containing no tetracycline.

### Example 5: Transplantation into nude mouse

g6 cells, g6 LAP cells, g6 LAP' cells, and g6 LAP" cells were cultured in the absence of tetracycline, and 10⁷ cells of each cell line were collected and washed twice with PBS. Five purchased nude mice (Bal b/c) were tested: 10⁷ of g6 cells were intraperitonially injected into 2 mice, and 10⁷ of g6 LAP cells, g6 LAP' cells or g6 LAP" cells were intraperitonially injected into the remaining 3 mice, respectively. After 30 days, all the mice were sacrificed and laparotomized. g6-transplanted mice had remarkable cancer formation and metastasis. In contrast, g6 LAP-, g6 LAP'-, or g6 LAP"-transplanted mice were found to have no cancer formation and metastasis. According to the above results, it was demonstrated that g6 LAP cells, g6 LAP' cells, and g6 LAP" cells, all having mutation-introduced LAP genes, lost cancer formation ability.

## Claims

1. A recombinant vector encoding LAP (transciptional activator) activity wherein expression of LIP activity is inhibited, and wherein expression of LIP activity is inhibited such that LIP does not down-regulate LAP activity.

2. The recombinant vector of claim 1 comprising a nucleotide sequence for part or all of the CCAAT/enhancer binding protein β (C/EBPβ) gene wherein said part or all of the C/EBPβ gene comprises a nucleotide sequence around the initiation codon, ATG, of the LIP transcription inhibitor protein, and wherein the nucleotide sequence comprises a mutation around the initiation codon, ATG, of said LIP transcription inhibitor protein.

3. The recombinant vector of claim 2 wherein said mutation is a substitution of ATG with a codon encoding another amino acid, wherein the substituted codon is not TTG.

4. The recombinant vector of any of claims 3 wherein said mutation is a substitution of ATG with a codon encoding an amino acid from the group consisting of Ala, Gly, and Pro.

5. The recombinant vector of any of claims 4 wherein said amino acid is Ala.

6. The recombinant vector of any of claims 4 wherein said amino acid is Gly.

7. The recombinant vector of any of claims 4 wherein said amino acid is Pro.

8. The recombinant vector of claim 3 wherein said amino acid is Arg.

9. The recombinant vector of any of claims 1 to 8 wherein said recombinant vector is a viral vector.

10. The recombinant viral vector of claim 9 wherein the vector is selected from the group consisting of a retrovirus vector, an adenovirus vector, and an adeno-associated virus vector.

11. A composition comprising the recombinant vector of any of claims 1 to 10.

12. The composition of claim 11 further comprising a pharmaceutically acceptable carrier.

13. A variant LAP polypeptide comprising a substitution of the initiating codon, ATG, of LIP with another codon, wherein the substituted codon is not TTG.

14. The variant LAP polypeptide of claim 13, wherein said substituted codon is not a translational stop codon.

15. An isolated nucleic acid encoding a variant LAP polypeptide of any one of claims 13 to 14.

16. A host cell comprising the recombinant vector of any of claims 1 to 10.

17. A host cell comprising the variant polypeptide of anyone of claims 13 to 14.

18. A host cell comprising the isolated nucleic acid of claim 15.

19. A viral particle comprising the recombinant viral vector of any of claims 9 to 10.

20. An oligonucleotide comprising a nucleotide sequence of about 10 to about 100 nucleotides in length from around the initiation codon, ATG, of LIP (transcription inhibitor protein) in the nucleotide sequence of a C/EBPβ gene, or a complementary sequence thereof, wherein said oligonucleotide, or a complementary sequence thereof, is capable of reducing LIP expression.

21. The oligonucleotide sequence of claim 20 wherein said oligonucleotide sequence is from about 10 to 80 nucleotides in length.

22. The oligonucleotide sequence of any of claims 20 to 21 wherein said oligonucleotide sequence is from about 15 to about 50 nucleotides in length.

23. A composition comprising the oligonucleotide of any one of claims 20 to 22.

24. The composition of claim 23 further comprising a pharmaceutically acceptable excipient.

25. A method for modifying the ratio of the expression level of LAP (transcription activator) to LIP (transcription inhibitor protein) in a cell, comprising contacting the cell with a recombinant vector of any one of claims 1-10, or a variant LAP polypeptide of anyone of claims 13 to 14 or an oligonucleotide of any one of claims 20 to 22 under suitable conditions.

26. The method of claim 25 wherein said cell is a cancer cell.

27. The method of any one of claims 25 to 26 wherein said cell is a breast cancer cell or a liver cancer cell.

28. A method of screening whether an agent modulates the ratio of the expression level of LAP (transcription activator) to LIP (transcription inhibitor protein) which are expressed from a C/EBPβ gene comprising the step of contacting the C/EBPβ gene with said agent and measuring the ratio of LAP expression to LIP expression.

29. The method of claim 28 wherein said agent is a low molecular weight compound.

30. The method of claim 28 wherein said agent is an extract from a naturally occurring substance.

31. The method of claim 28 wherein the ratio is measured by Northern blot.

32. The method of claim 28 wherein a mammalian cell comprises said C/EBP 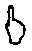 gene.

33. The method of claim 28 further comprising identifying an agent that increases the expression level of LAP in a cell.

34. The method of claim 28 further comprising identifying an agent that decreases the expression level of LIP in a cell.

35. The method of claim 28 wherein the ratio is measured by PCR.

36. A method for treating and/or ameliorating the symptoms of a disease and/or condition associated with an abnormal ratio of the expression level of LAP (transcription activator) to LIP (transcription inhibitor protein) in an individual comprising administering to the individual a recombinant vector of anyone of claims 1 to 10, or an oligonucleotide of anyone of claims 20 to 22, or a LAP polypepide of anyone of claims 13 to 14.

37. The method of claim 36 wherein said individual is subject to cancer.

38. The method of claim 37 wherein said cancer is breast cancer or liver cancer.

39. The use of a recombinant vector of anyone of claims 1 to 10 in the manufacture of a medicament for the treatment and/or amelioration of symptoms of cancer.

40. The use of a variant LAP polypeptide of anyone of claims 13 to 14 in the manufacture of a medicament for the treatment and/or amelioration of symptoms of cancer.

41. The use of an oligonucleotide of anyone of claims 20-22 in the manufacture of a medicament for the treatment and/or amelioration of symptoms of cancer.
